# EUROPEAN PATENT SPECIFICATION

(11) **EP 1 816 972 B1**
(45) Date of publication and mention of the grant of the patent: **07.09.2016**
(21) Application number: 05848903.0
(22) Date of filing: 01.12.2005
(51) Int. Cl.: A61B 90/98, B65D 23/00, B65D 23/14

(54) **RADIO FREQUENCY IDENTIFICATION FOR MEDICAL DEVICES**
RADIOFREQUENZIDENTIFIKATION FÜR MEDIZINISCHE VORRICHTUNGEN
IDENTIFICATION RADIOFREQUENCE DE DISPOSITIFS MEDICAUX

(30) Priority: 02.12.2004 US 632679 P
(43) Date of publication of application: 15.08.2007
(73) Proprietor: Smith & Nephew, Inc, Memphis, TN 38116 (US)
(72) Inventor: DONATI, Ralph, Germantown, TN 38139 (US); TROUTMAN, Randall, Cordova, TN 38138 (US)
(74) Representative: Smith & Nephew
(86) International application number: PCT/US2005/043957
(87) International publication number: WO 2006/060781

(56) References cited:
- US-A- 5 294 413
- US-A1- 2002 143 320
- US-A1- 2002 188 259
- US-A1- 2003 052 788
- US-A1- 2004 008 123

## Description

This application claims the benefit of U.S. Provisional Application No. 60/632,679, filed 12/02/2004.

### RELATED FIELDS

Embodiments of the present invention relate to systems and methods for identifying sterilized medical devices located within a sterilization case that is sealed with a wrap in preparation for a surgical procedure.

### BACKGROUND

Hospitals, instrument management companies, and steritization companies often have difficulty tracking and managing surgical instruments as they pass through the purview of various parties in the supply chain. For instance, instruments are sometimes misplaced, which may remain unknown until the instruments are removed from the sterilization case and the wrap in the sterile field of the operating room prior to surgery. If one or more instruments needed for the surgery are missing, personnel must open another sealed plastic wrap to retrieve the missing instruments or locate a replacement within the hospital. Personnel associated with surgical preparation sometimes need to open several sealed packets before they obtain a complete set of necessary instruments or spend time searching the facility for a replacement. Furthermore, the remaining instruments in the second sterilization case and those opened thereafter must be sterilized again.

- Various methods of counting the surgical instruments are known, but none are particularly efficient. One method involves a person physically counting each instrument on the surgical instrument tray and then comparing the result to an information sheet that provides an inventory list of instruments used in a particular surgery. This count is usually performed close to, but before, the scheduled surgery while the patient is either already on the surgical table or on their way to the surgical room. If there is a discrepancy between the count and inventory list, the person counting or their assistant must quickly determine which instrument is missing and where a suitable replacement may be located before the surgical procedure begins.

Such tracking processes require time to count the instruments manually, determine if there is a discrepancy between the count and the inventory list, and locate a replacement instrument. This time is costly to both the instrument company and hospital and could needlessly delay surgery. In addition, there is a likelihood for human error in counting the instruments and comparing the count to the inventory list. Furthermore, the correct instruments for a particular surgical procedure may still be missing during surgery, forcing the surgeon to use a closely related, but incorrect, instrument to perform the procedure. Manually tracking medical instruments also requires that the surgical instruments be removed from the sealed sterilization case before they are counted and compared to the inventory list for discrepancies.

Another method to inventory and manage medical instruments involves attaching radio frequency identification (RFID) tags to the surgical instruments and providing a reader that obtains information associated with the particular medical instrument through radio frequency. RFID tags typically comprise an electronic circuit placed on small substrate materials. The electronic circuits contain encoded data and transmit or respond (actively or passively) with encoded or identifiable data as a radio frequency signal or a signature when an interrogation radio frequency signal causes the electronic circuit to transmit or respond (whether actively or passively). Some RFID tags are able to have their data modified by an encoded radio signal.

A reader is a radio frequency emitter/receiver or interrogator. In accordance with general RFID tag methodology, the reader interrogates RFID tags that are within its range by emitting radio frequency waves at a certain frequency. Each tag may respond to a unique set of interrogation frequencies. An RFID tag typically responds to an interrogation by emitting or responding with coded or identification information as a radio frequency signal or signature and this signal or signature (whether actively or passively) is detected by the reader. The reader is in electrical communication with a computer system having a database of information about the inventory. After detecting the radio frequency signal from the RFID tag, the reader causes the computer system to change the data in the database to account for the presence of a particular inventory item.

An RFID tag system has several advantages over manual counting management methods. For instance, an RFID tag system is capable of quickly performing an inventory of a large group of items by successively reading a tag associated with each item without requiring the user to perform multiple steps.

Known RFID tag systems have been used to manage medical instrument locations prior to and during surgery. For instance, the surgical tray table may be scanned prior to the surgery to ensure that all instruments needed for the procedure are present. Prior to completing the surgery, the surgical tray table may be scanned again to ensure that instruments are located on the tray table instead of inside the patient. Some RFID tag systems describe scanning the surgical cavity of the patient to check for the presence of any instruments prior to completing the surgery.

Previous and current RFID tag systems used to manage and inventory medical instruments require that surgical personnel break the sterilization seal of a group of instruments and remove the instruments from the packet before the reader reads medical instruments. This is because instruments are typically contained within a metallic sterilization case and the sterilization case prevents electromagnetic energy, such as a radio frequency signal, from entering or leaving the case. Thus, an RFID reader is unable to communicate with the RFID tags located inside the sterilization case and the instruments must be removed from the case, including breaking the sealed wrap, in order for the reader to determine the inventory of a particular group of instruments. If, after reading the removed medical instruments, the medical instrument group does not include an instrument necessary for the particular surgical procedure, hospital or medical instrument company representatives must break another sealed packet, remove the instruments from the case and read or interrogate the RFID tags of that group to find the instrument necessary to complete the first instrument group.

A medical instrument inventory and management system that allows personnel to read data regarding the individual medical instruments contained within a sealed sterilization case would decrease the time necessary to locate a particular instrument. In addition, determining the presence of particular medical instruments inside a sealed sterilization case could decrease the time and cost of preparing for a surgical procedure since breaking a second and additional sealed packets requires another cleaning, decontamination, and sterilization process. Furthermore, personnel could read the inventory of several packets and select the one with the correct instrument group for a particular medical procedure. US20031052788, from which the preamble of claim 1 is known, discloses a meditation tracking system which includes a medicament or medical instrument associated with a smart tag e.g. a RFID tag and an apparatus comprising a decoder for reading information from the smart tag and a processor for storing information from the smart tag.

### SUMMARY

Accordingly, the present invention relates generally to systems and methods for identifying medical instruments without breaking a sealed wrap surrounding the sterilization case and instruments and managing and taking an inventory of medical instruments.

An RFID tag is affixed to each medical instrument prior to the sterilization procedure. Each RFID tag contains, or is associated with, information related to the particular medical instrument to which it is attached. The medical instruments, along with their respective attached RFID tags, are placed inside a sterilization case which is topically metal or another material that is not conducive to allowing electromagnetic energy, such as radio frequency signals, from entering or leaving the case. A plastic covering may then be wrapped around the sterilization case and sealed to prevent any contaminants from entering the sterilization case. The instrument packet enters a sterilization process to remove contaminants from the medical instruments. After the sterilization process, the instruments remain in the sealed sterilization case.

Prior to the surgical procedure, a reader scans the instruments contained in the sealed sterilization case. A reader is brought within proximity of the instrument packet and transmits one or more signals that are received by RFID tags contained within the packet. The RFID tags respond actively or passively, such as by transmitting or evidencing a responsive signature with encoded data or other identifying data. The sterilization case is configured to allow electromagnetic energy, such as radio frequency signals, to enter and leave the case. Configurations could include holes, slits, or any other type and size of opening or openings in at least one side of the sterilization case or the sterilization case may be made from a material, such as plastic, paper products, wood, cloth, vinyl, metal or leather, that allows radio frequency signals to enter and leave the case. The reader compares this data set to a database either contained within the reader or contained within another device (i.e. a computer or network) that is in communication with the reader. Information in the database corresponding to the encoded data or identifying information is then displayed to the user on an interface contained within the reader or within a separate device. This information could include the identification of the medical instrument or any other aspect regarding each individual medical instrument or the medical instrument packet.

If the displayed results indicate that all instruments needed for the particular procedure are located inside the packet, the user may break the sealed wrap and remove the medical instruments from the sterilization case. If the displayed results indicate that some instruments are missing from the packet, another packet may be quickly selected and read to determine if it contains all the necessary instruments for a particular surgical procedure. In the alternative, the user may select and read a second packet to determine if the instruments missing in the first read packet are located in the second read packet. Based on the information obtained by reading the packets, the appropriate packet may be selected and its sealed wrap broken for a particular procedure. In the alternative, the user may quickly determine the two packets on which to break the sealed wrap in order to complete a medical instrument set for a particular surgical procedure.

### STATEMENT Of THE INVENTION

According to the invention, there is: A system for identifying medical instruments in a sealed sterilization case according to claim 1.

According to the invention, there is also provided a method for identifying at least one medical instrument attached or associated with a radio frequency identification tag in a sealed sterilization case with a reader that obtains information from the radio frequency identification tag. according to claim 10.

Also disclosed herein, but not forming part of the invention, is a sterilization case is composed of a material that is configured to allow radio frequency signals to enter and leave the case.

In one embodiment, there is provided:
a system for identifying medical instruments in a sealed sterilization case according to the system above, wherein the radio frequency identification tag associated with the instrument is embedded inside the instrument.

In one embodiment, there is provided:
a system for identifying medical instruments in a sealed sterilization case according to the system above, wherein the radio frequency identification tag associated with the instrument is attached on the outside surface of the medical instrument.

In one embodiment, there is provided: a system for identifying medical instruments in a sealed sterilization case according to the system above, wherein each of the radio frequency identification tags is a passive device.

In one embodiment, there is provided: a system for identifying medical instruments in a sealed sterilization case according to the system above, wherein each of the radio frequency identification tags is an active device.

In one embodiment, there is provided: a system for identifying medical instruments in a sealed sterilization case according to the system above, wherein the reader is electrically connected to a microprocessor.

In one embodiment, there is provided: a system for identifying medical instruments in a sealed sterilization case according to the system above, wherein the reader is in wireless communication with a microprocessor.

In one embodiment, there is provided: a system for identifying medical instruments in a sealed sterilization case according to the system above, wherein the information includes at least one of the following:
the identification of the medical instrument to which the radio frequency identification tag is associated with;
the surgical technique associated with the medical instrument to which the radio frequency identification tag is associated with; and
the manufacturing history of the medical instrument to which the radio frequency identification tag is associated with.

In one embodiment, there is provided a system for identifying medical instruments in a sealed sterilization case according to the system above, further characterized in that a case radio frequency identification tag attached or associated with the outside of the sterilization case, wherein the case radio frequency obtains information, via radio frequency, from the radio frequency tags associated with the medical instruments and communicates the information via radio frequency to the reader.

Also disclosed herein, but not forming part of the invention, is a method for identifying at least one medical instrument attached or associated with a radio frequency identification tag in a sealed sterilization case in the method above, further characterized in that the sterilization case is composed of a material that is configured to allow radio frequency signals to enter and leave the case.

In one embodiment, there is provided: a method for identifying at least one medical instrument attached or associated with a radio frequency identification tag in a sealed sterilization case in the method above, wherein the radio frequency identification tag associated with the instrument is embedded inside the instrument.

In one embodiment, there is provided: a method for identifying at least one medical instrument attached or associated with a radio frequency identification tag in a sealed sterilization case in the method above, wherein the radio frequency identification tag associated with the instrument is attached on the outside surface of the medical instrument.

In one embodiment, there is provided: a method for identifying at least one medical instrument attached or associated with a radio frequency identification tag in a sealed sterilization case in the method above, wherein each of the radio frequency identification tags is a passive device.

In one embodiment, there is provided: a method for identifying at least one medical instrument attached or associated with a radio frequency identification tag in a sealed sterilization case in the method above, wherein each of the radio frequency identification tags is an active device.

In one embodiment, there is provided: a method for identifying at least one medical instrument attached or associated with a radio frequency identification tag in a sealed sterilization case in the method above, wherein the reader is electrically connected to a microprocessor.

In one embodiment, there is provided: a method for identifying at least one medical instrument attached or associated with a radio frequency identification tag in a sealed sterilization case in the method above, wherein the reader is in wireless communication with a microprocessor.

In one embodiment, there is provided a method for identifying at least one medical instrument attached or associated with a radio frequency identification tag in a sealed sterilization case in the method above, wherein the information includes at least one of the following:
the identification of the medical instrument to which the radio frequency identification tag is associated with;
the surgical technique associated with the medical instrument to which the radio frequency identification tag is associated with; and
the manufacturing history of the medical instrument to which the radio frequency identification tag is associated with.

In one embodiment, there is provided a method for identifying at least one medical instrument attached or associated with a radio frequency identification tag in a sealed sterilization case in the method above, further characterized in that a case radio frequency identification tag attached or associated with the outside of the sterilization case, wherein the case radio frequency obtains information, via radio frequency, from the radio frequency tags associated with the medical instruments and communicates the information via radio frequency to the reader.

### BRIEF DESCRIPTION OF THE FIGURES

FIG. 1 shows a sterilization case and RFID tag reader according to one embodiment of the invention.
FIG. 2 shows a sealed wrap over a sterilization case and RFID tag reader according to one embodiment of the invention.
FIG. 3 shows a second medical instrument packet and RFID tag reader according to one embodiment of the invention.
FIG. 4 shows a medical instrument with an RFID tag located on the outside surface of the instrument according to one embodiment of the invention.
FIG. 5 shows a medical instrument with an RFID tag embedded in the instrument according to one embodiment of the invention.
FIG. 6 shows a reader electrically connected to a computer system according to one embodiment of the invention.

### DETAILED DESCRIPTION OF THE FIGURES

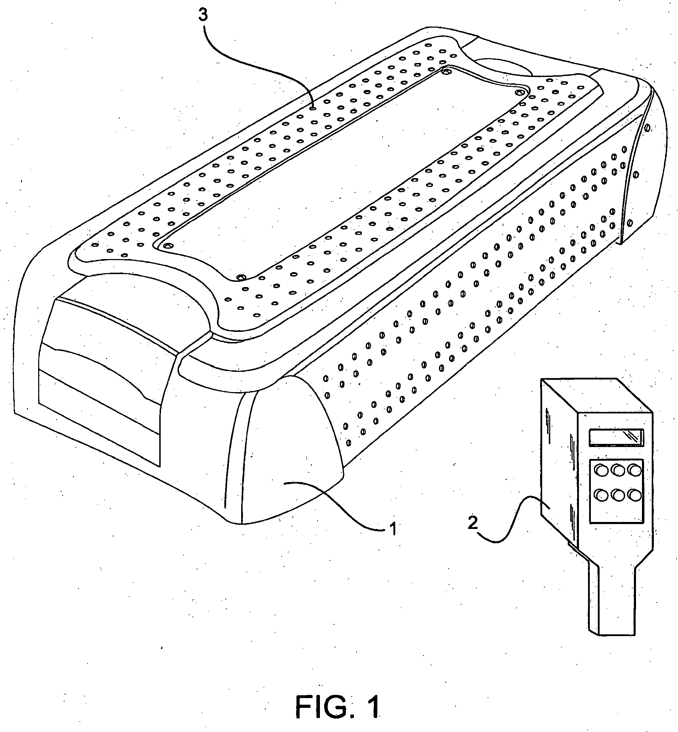

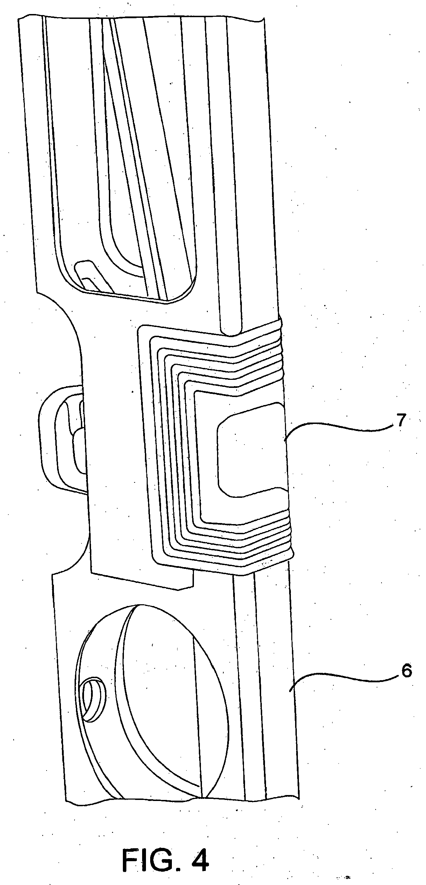

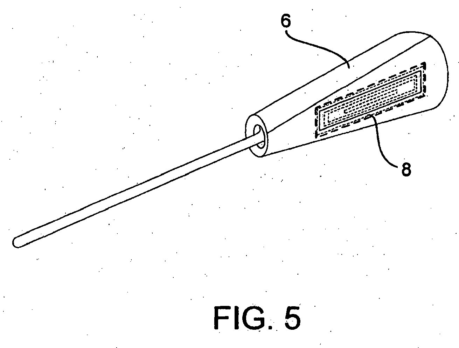

Referring initially to **FIG. 1**, illustrated is a system for identifying medical devices according to one embodiment of the invention. Included in this embodiment is a sterilization case **1** and an RFID reader **2** configured to communicate with RFID tags. The sterilization case **1** may contain various medical instruments, such as medical instruments **6** shown in **FIGs. 4** and **5****,** and RFID tags, such as RFID tags **7** and **8** shown in **FIGs. 4** and **5****.** It should be understood that other types of medical instruments and RFID tag configurations may be used with the several embodiments of the present invention. RFID tags **7** and **8** are attached to, associated with, or embedded in the medical instruments **6.** **FIG. 4** shows one embodiment of the invention where an RFID tag **7** is associated or attached to medical instrument **6**. **FIG. 5** shows another embodiment of the invention where an RFID tag **8 is** embedded in the medical instrument **6**.

In some embodiments, RFID tags **7** and **8** are attached to the medical instruments **6** by an adhesive substance such as glue, paste, gum, epoxy resin, tape, bonding agent, or any other type of adhesive that will attach the RFID tags **7** and **8** to the medical instrument **6**. In other embodiments, RFID tags **7** and **8** are attached to the medical instruments **6** by a mechanical device such as a clip, fastener, clasp, pin, screw, or any other device that will mechanically associate an RFID tag to a medical instrument. In other embodiments, RFID tags **7** and **8** may be attached to the medical instruments **6** by molding or otherwise, including during manufacture of medical instruments **6** or otherwise.

Referring again to **FIG. 1**, in some embodiments, the sterilization case **1** may be made from steel, aluminum, titanium, plastic or any other appropriate material that will provide containment for sterilization purposes. In some embodiments, the sterilization case **1** may include at least one side with at least one opening **3**, such as holes, slits, or any other type and size of opening or openings in at least one side of the sterilization case shaped and sized to allow RFID tags **7** and **8** in the sterilization case **1** to respond to a reader **2.** In other embodiments, at least one side of the sterilization case **1** may be made from materials such as plastic, paper products, wood, cloth, vinyl, leather, or any other appropriate material that allows radio frequency signals to enter and leave the case.

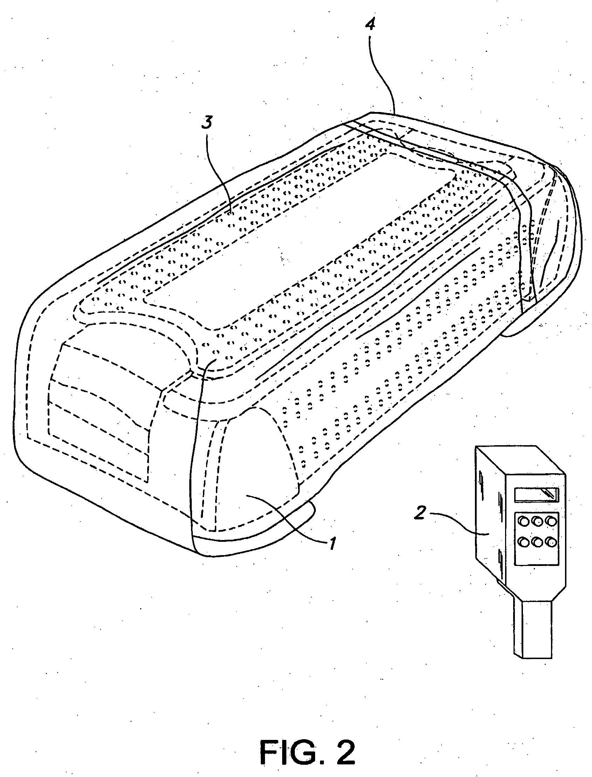

Referring now to **FIG. 2****,** illustrated is another alternative embodiment of the present invention. Included in this embodiment is a reader **2** and a sealed wrap **4** surrounding a sterilization case **1** with at least one opening **3**. The sealed wrap **4** may be composed of assorted materials such as various combinations of plastic, paper, polyethylene film, polyester film, foil, laminate material, polypropylene, polyolefin, polymusum, or any other material that may be used to wrap the sterilization case for sterilization purposes.

RFID tags **7** and **8** of certain embodiments of the invention, associated with medical instruments **6** and located inside the sterilization case **1,** have the capability of transmitting or responding with encoded data or a signature when they are interrogated by a reader **2.** The RFID tags of the preferred embodiment are passive, sin that they do not contain an independent energy source but must depend on the radio frequency signal from the reader to provide its response or signature. An alternative embodiment of the present invention includes active RFID tags that contain an independent energy source, such as a battery or other means, so that the RFID tag can actively transmit a signal or information.

The preferred embodiment of the reader **2** is shown as a handheld device capable of transmitting a signal via radio frequency to the RFID tags **7** and **8** and receiving encoded data or RFID tag signatures via radio frequency from the RFID tags **7** and **8**. The preferred embodiment of the reader **2** also has the capability of outputting the received data onto a viewable interface or to a computer via electrical or wireless connection. It should be understood by those with skill in the art that the reader **2** may take any stationary or movable form with the function of reading RFID tags. For example, in an alternative embodiment of the present invention the reader **2** is a mat and the sterilization case **1** containing the medical instruments **6,** may be placed on the mat. The reader **2** then reads the RFID tags attached to the medical devices.

When the sterilization case **1** is made from steel, aluminum, titanium, or any type of metal, radio frequency communication between an RFID tag **7** and **8** and a reader **6** may be greatly attenuated and the presence of metal may prevent all communication. The sterilization case, however, may be modified to allow radio frequency communication between the RFID tags **7** and **8** and reader **2.** Modifications to the sterilization case may include openings **3** such as holes, slits, or any other type and size of opening in at least one side of the sterilization case. In other embodiments the sterilization case **1** may be made from a material, such as plastic, paper products, wood, cloth, vinyl, metal or leather, that allows radio frequency signals to enter and leave the case. In still other embodiments, a master RFID tag or reader may be attached to the outside of the sterilization case to collect information from inside the case and communicate the information to an external reader.

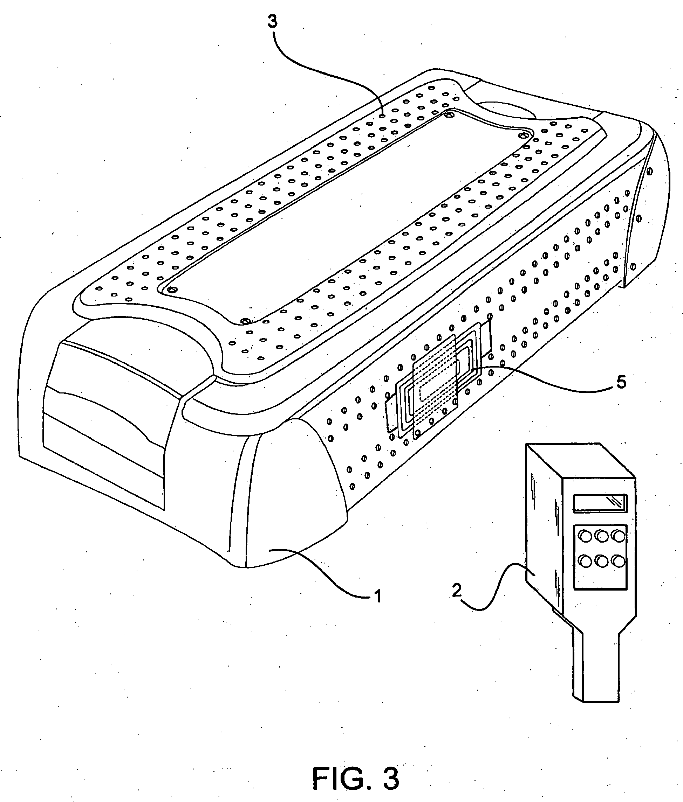

Referring now to **FIG. 3****,** illustrated is another alternative embodiment of the present invention. Included in this embodiment is a sterilization case **1** and RFID tag reader **2.** The sterilization case **1** contains RFID tags attached to medical instruments. The sterilization case **1** also contains a case RFID tag **5** attached on the outside of at least one of the sterilization case's **1** surfaces. In some embodiments, the case RFID tag **5** has the capability of receiving a signal from the reader **2** and then transmitting or responding with encoded data or identifying information to the reader **2.** In other embodiments, the case RFID tag **5** may communicate through radio frequency signals to the RFID tags associated with the medical instruments located inside the sterilization case **1.** The case RFID tag **5** then communicates the information received from the RFID tags inside the case to the reader **2.**

In certain embodiments of the present invention, the type of data transmitted by the RFID tags **7** and **8** and case RFID tag **5** to the reader **2** may include the identification of the medical instruments **6** to which the RFID tags **7** and **8** are attached, the contents of the entire sterilization case **1,** the surgical technique associated with a particular medical instrument **6** or group of medical instruments contained within the sterilization case **1,** surgical implants with which the instrument **6** is to be used, the manufacturing history of a particular medical instrument **6,** how many times the instrument **6** has been sterilized, or any other relevant data associated with the instruments, group of instruments, or case. Alternatively, the RFID tags **7** and **8** may respond with a signal or signature that keys or correlates to such information in a database in the computer system or on a network such as the Internet or a local network. One may see that a number of different types of data may be conveyed with or keyed to information conveyed using RFID tags **7** and **8** and case RFID tag **5**. In the preferred embodiment, the RFID tags **7** and **8** transmit or respond with data that corresponds to the identification of a particular medical instrument **6** and the case RFID tag **5** transmits data corresponding to the identification and contents of a particular sterilization case **1**.

Referring now to **FIG. 4****,** illustrated is the preferred embodiment of an RFID tag **7** attached to a medical instrument **6.** The RFID tag **7** is attached on the outside surface of the medical instrument **6.** Various methods may be utilized to attach the RFID tag **7** on the outside surface of the medical instrument **6.** For example, the RFID tag **7** may be attached on the outside surface of the medical instrument **6** through any adhesive or mechanical device. In some embodiments, the adhesive substance may be glue, paste, gum, epoxy resin, tape, bonding agent, or any other type of adhesive that will attach an RFID tag **7** to a medical instrument **6.** In other embodiments, RFID tags **7** are associated to the medical instrument **6** by a mechanical device. The mechanical device may be a clip, fastener, clasp, pin, screw, or any other device that will mechanically associate an RFID tag to a medical instrument.

Referring now to **FIG. 5****,** illustrated is one embodiment of a RFID tag **8** attached to a medical instrument **6.** In this particular embodiment, the RFID tag **8** is embedded inside the medical instrument **6** and is not viewable from the surface of the medical instrument **6**. Various procedures may be utilized to embed the RFID tag **8** into medical instrument **6** such as removing a portion of the medical instrument and replacing the portion with an RFID tag **8.** Other procedures apparent to those of ordinary skill in the art may be utilized to embed the RFID tag **8.**

Referring again to **FIG. 2****,** to inventory and manage medical instruments while they are in a sealed wrap **4,** the user begins by waving the reader **2** in proximity to the sealed and wrapped sterilization case **1**. This distance between the reader **2** and sterilization case **1** may be as little as 5 millimeters to as much as several feet or more. In an alternative embodiment of the present invention, the reader **2** may be stationary while the user locates the sealed and wrapped sterilization case **1** in proximity to a stationary reader **2.** The reader **2** transmits a radio frequency signal interrogating the RFID tags associated with medical instruments inside the sterilization case **1**. The RFID tags respond to the interrogation by transmitting a radio frequency signal or responding with a signature containing encoded or indicative data. The reader **2** receives these signals and sends them to a computer and/or correlates them to a database contained within the reader **2** or elsewhere such as on a network.

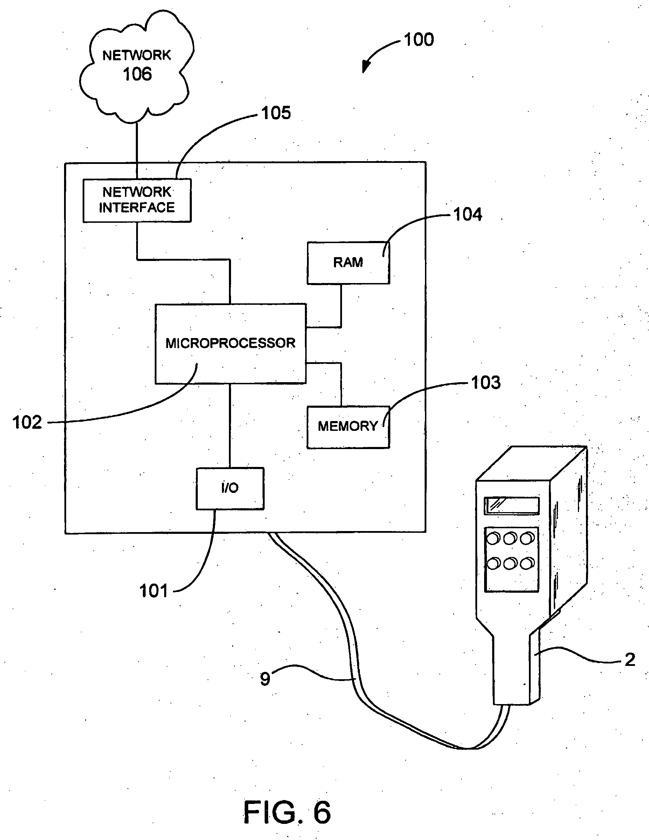

Referring now to **FIG. 6****,** in the preferred embodiment of the present invention, the reader **2** sends the encoded or indicative data to a computer system **100** via electrical signals along a cable **9.** In an alternate embodiment of the present invention, the reader **2** communicates the encoded or indicative data received from the RFID tags via wireless connection to a computer system **100.** In some embodiments, the computer system **100** includes an input/output **101,** microprocessor **102,** memory device **103**, random access memory **104,** and network interface **105**. The input/output **101** takes in the information from the reader and sends the information to the microprocessor **102**. The microprocessor **102** compares the information with a database in the memory **103** and/or random access memory **104**. The microprocessor **102** then displays the results onto an interface, such as a monitor, that may be viewed by a user, sends the information in the memory **103** for storage, and/or sends the results to a network interface **105** that is in communication with a network **106**. The network **106** may include a plurality of computer systems, an Internet, a local area network, or any type of network systems capable of processing or transferring data.

In an alternative embodiment the reader **2** comprises a database and viewable interface. The reader **2** compares the encoded data with the database and the results on the viewable interface.

The user may utilize the results displayed on the interface to determine whether all necessary instruments for a particular surgical procedure are contained within the packet, if some necessary instruments are missing to locate them in another instrument packet, or to locate a packet that contains all necessary instruments.

One example in accordance with the present invention is as follows. In preparation for a surgical procedure, such as a total knee replacement, members of medical device central processing sterilize the medical instruments to be used. A variety of medical instruments may be sterilized, such as a cutting block, fin stem punch, femoral trial, and patella clamp.

Prior to sterilization, a passive RFID tag, such as those manufactured or supplied by Danby, TTP, QuintiQ, or Precimed may be attached to each medical instrument. The RFID tag is preferably attached by an instrument management company to the medical instruments with an epoxy adhesive, but may be attached immediately prior to sterilization by a clip or some other mechanical method.

The medical instruments, with an RFID tag attached to each one, are placed inside a sterilization case, such as model number 7112-9401/9402/9400 manufactured by Smith and Nephew. The sterilization case is wrapped in a plastic wrap, such as Kimberly-Clark 600 sterilization pouch or sterilization wrapper, and the wrap is then sealed.

The sealed sterilization case containing the medical instruments is placed in the sterilization pouch and the pouch is sealed. The completed assembly is then placed into an autoclave and subjected to a medical autoclave sterilization process. The sterilization case is removed then from the autoclave and placed on a shelf.

The wrapped instrument cases are taken to a central storage location. When the case is scheduled, they are taken to a staging area or up to the operating room. Just prior to surgery, a medical instrument sales representative, hospital employee, or nurse scans the instrument packets in central processing, a staging area, or in the operating room with an RFID reader. The reader is preferably a handheld reader, but may also be a mat reader, a stationary reader, or any other reader disclosed herein. In a particularly preferred embodiment, the reader is handheld and is scanned over the wrapped sterilization case prior to the sealed outer wrap being broken.

The information obtained from the RFID tag is sent, preferably through wireless connection, to a computer or handheld computing device, such as a Hewtet-Packard iPAQ or a network. In an alternative embodiment, the information is displaced on a screen located on the reader. The output may show, for example, a sterilization case list, the contents of the case, the part numbers necessary for a procedure, which instruments are missing, or any information relevant to medical instrument inventory and management. If the interface indicates that all instruments necessary for the procedure are present, then the sealed plastic wrap is broken, the instruments are removed from the sterilization case, and laid on a tray table in the operating room. If the interface indicates that all instruments necessary for the procedure are present, then the person scanning would notify someone assisting in the surgery that the packet is not complete and that another packet is read to supplement or replace the first packet.

## Claims

1. A system for identifying medical instruments in a sterilization case comprising: (a) at least one radio frequency identification tag (7, 8); (b) at least one medical instrument (6) contained in a case (1) and attached to the radio frequency identification tag; (c) a reader (2) adapted to obtain information, via radio frequency, from said radio frequency identification tag; wherein
the case and the RFID tags are adapted to permit the reader to obtain information, via radio frequency, when the medical instrument is container in the case, **characterised in that** the case is a sterilization case surrounded by a sealed wrap (4), and **in that** at least one surface of the sterilization case has at least one opening (3) adapted in size or shape to allow radio frequency signals to enter and leave the case.

2. A system for identifying medical instruments in a sterilization case according to claim 1, wherein the radio frequency identification tag associated with the instrument is embedded inside the instrument.

3. A system for identifying medical instruments in a sterilization case according to claim 1, wherein the radio frequency identification tag associated with the instrument is attached on the outside surface of the medical instrument.

4. A system for identifying medical instruments in a sterilization case according to claim 1, wherein each of the radio frequency identification tags is a passive device.

5. A system for identifying medical instruments in a sterilization case according to claim 1, wherein each of the radio frequency identification tags is an active device.

6. A system for identifying medical instruments in a sterilization case according to claim 1, wherein the reader is electrically connected to a microprocessor (102).

7. A system for identifying medical instruments in a sterilization case according to claim 1, wherein the reader is in wireless communication with a microprocessor (102).

8. A system for identifying medical instruments in a sterilization case according to claim 1, wherein the information includes at least one of the following:
the identification of the medical instrument to which the radio frequency identification tag is associated with;
the surgical technique associated with the medical instrument to which the radio frequency identification tag is associated with; and
the manufacturing history of the medical instrument to which the radio frequency identification tag is associated with.

9. A system for identifying medical instruments in a sterilization case according to claim 1, further **characterized in that** a case radio frequency identification tag (5) attached or associated with the outside of the sterilization case, wherein the case radio frequency obtains information, via radio frequency, from the radio frequency tags associated with the medical instruments and communicates the information via radio frequency to the reader.

10. A method for identifying at least one medical instrument (6) attached or associated with a radio frequency identification tag (7, 8) in a case (1) with a reader (2) that obtains information from the radio frequency identification tag wherein the method comprises:
providing the case configured to allow radio frequency signals to enter and leave the case and inserting each medical instrument and radio frequency identification tag into the case; and
using the reader to obtain information, via radiofrequency, when each medical instrument is contained in the case, **characterized in that** the case is a sterilization case surrounded by a sealed wrap (4), and
at least one surface of the sterilization case has at least one opening (3) adapted in size or shape to allow radio frequency signals to enter and leave the case.

11. A method for identifying at least one medical instrument attached or associated with a radio frequency identification tag in a sterilization case in claim 10, wherein the radio frequency identification tag associated with the instrument is embedded inside the instrument.

12. A method for identifying at least one medical instrument attached or associated with a radio frequency identification tag in a sterilization case in claim 10, wherein the radio frequency identification tag associated with the instrument is attached on the outside surface of the medical instrument.

13. A method for identifying at least one medical instrument attached or associated with a radio frequency identification tag in a sterilization case in claim 10, wherein each of the radio frequency identification tags is a passive device.

14. A method for identifying at least one medical instrument attached or associated with a radio frequency identification tag in a sterilization case in claim 10, wherein each of the radio frequency identification tags is an active device.

15. A method for identifying at least one medical instrument attached or associated with a radio frequency identification tag in a sterilization case in claim 10, wherein the reader is electrically connected to a microprocessor.

16. A method for identifying at least one medical instrument attached or associated with a radio frequency identification tag in a sterilization case in claim 10, wherein the reader is in wireless communication with a microprocessor (102).

17. A method for identifying at least one medical instrument attached or associated with a radio frequency identification tag in a sterilization case in claim 10, wherein the information includes at least one of the following:
the identification of the medical instrument to which the radio frequency identification tag is associated with;
the surgical technique associated with the medical instrument to which the radio frequency identification tag is associated with; and
the manufacturing history of the medical instrument to which the radio frequency identification tag is associated with.

18. A method for identifying at least one medical instrument attached or associated with a radio frequency identification tag in a sterilization case in claim 10, further **characterized in that** a case radio frequency identification tag attached or associated with the outside of the sterilization case, wherein the case radio frequency obtains information, via radio frequency, from the radio frequency tags associated with the medical instruments and communicates the information via radio frequency to the reader.

## Patentansprüche

1. Ein System zur Identifikation medizinischer Instrumente in einem Sterilisationsgehäuse, beinhaltend: (a) mindestens eine Radiofrequenzidentifikationsmarkierung (7, 8); (b) mindestens ein in einem Gehäuse (1) enthaltenes und an der Radiofrequenzidentifikationsmarkierung befestigtes medizinisches Instrument (6); (c) ein zum Erhalten von Informationen via Radiofrequenz von der Radiofrequenzidentifikationsmarkierung angepasstes Lesegerät (2); wobei das Gehäuse und die RFID-Markierungen angepasst sind, um dem Lesegerät zu gestatten, Informationen via Radiofrequenz zu erhalten, wenn das medizinische Instrument in dem Gehäuse enthalten ist, **dadurch gekennzeichnet, dass** das Gehäuse ein Sterilisationsgehäuse ist, umgeben von einer versiegelten Umhüllung (4), und dadurch, dass mindestens eine Oberfläche des Sterilisationsgehäuses mindestens eine Öffnung (3) aufweist, die in Größe oder Gestalt angepasst ist, (um) Radiofrequenzsignalen zu erlauben, in das Gehäuse einzudringen und es zu verlassen.

2. System zur Identifikation medizinischer Instrumente in einem Sterilisationsgehäuse gemäß Anspruch 1, wobei die mit dem Instrument assoziierte Radiofrequenzidentifikationsmarkierung innerhalb des Instruments eingebettet ist.

3. System zur Identifikation medizinischer Instrumente in einem Sterilisationsgehäuse gemäß Anspruch 1, wobei die mit dem Instrument assoziierte Radiofrequenzidentifikationsmarkierung an der Außenoberfläche des medizinischen Instruments befestigt ist.

4. System zur Identifikation medizinischer Instrumente in einem Sterilisationsgehäuse gemäß Anspruch 1, wobei jede der Radiofrequenzidentifikationsmarkierungen eine passive Vorrichtung ist.

5. System zur Identifikation medizinischer Instrumente in einem Sterilisationsgehäuse gemäß Anspruch 1, wobei jede der Radiofrequenzidentifikationsmarkierungen eine aktive Vorrichtung ist.

6. System zur Identifikation medizinischer Instrumente in einem Sterilisationsgehäuse gemäß Anspruch 1, wobei das Lesegerät elektrisch mit einem Mikroprozessor (102) verbunden ist.

7. System zur Identifikation medizinischer Instrumente in einem Sterilisationsgehäuse gemäß Anspruch 1, wobei das Lesegerät sich in drahtloser Kommunikation mit einem Mikroprozessor (102) befindet.

8. System zur Identifikation medizinischer Instrumente in einem Sterilisationsgehäuse gemäß Anspruch 1, wobei die Informationen mindestens eins der Folgenden einschließen:
die Identifikation des medizinischen Instruments, mit dem die Radiofrequenzidentifikationsmarkierung assoziiert ist;
die mit dem medizinischen Instrument, mit dem die Radiofrequenzidentifikationsmarkierung assoziiert ist, assoziierte chirurgische Technik; und
die Herstellungsgeschichte des medizinischen Instruments, mit dem die Radiofrequenzidentifikationsmarkierung assoziiert ist.

9. System zur Identifikation medizinischer Instrumente in einem Sterilisationsgehäuse gemäß Anspruch 1, ferner **dadurch gekennzeichnet, dass** eine Gehäuse-Radiofrequenzidentifikationsmarkierung (5) an der Außenseite des Sterilisationsgehäuses befestigt oder damit assoziiert ist, wobei die Gehäuse-Radiofrequenz via Radiofrequenz Informationen von den mit dem medizinischen Instrument assoziierten Radiofrequenzmarkierungen erhält, und die Informationen via Radiofrequenz an das Lesegerät kommuniziert.

10. Ein Verfahren zur Identifikation von mindestens einem an einer Radiofrequenzidentifikationsmarkierung (7, 8) in einem Gehäuse (1) mit einem Lesegerät (2) befestigten oder damit assoziierten medizinischen Instrument (6), das Informationen von der Radiofrequenzidentifikationsmarkierung erhält, wobei das Verfahren Folgendes beinhaltet:
Bereitstellen des Gehäuses, das konfiguriert ist, um Radiofrequenzsignalen zu erlauben, in das Gehäuse einzudringen und es zu verlassen, und Einführen jedes medizinischen Instruments und jeder Radiofrequenzidentifikationsmarkierung in das Gehäuse; und
Verwenden des Lesegeräts, um via Radiofrequenz Informationen zu erhalten, wenn jedes medizinische Instrument in dem Gehäuse enthalten ist, **dadurch gekennzeichnet, dass** das Gehäuse ein durch eine versiegelte Umhüllung (4) umgebenes Sterilisationsgehäuse ist, und
wobei mindestens eine Oberfläche des Sterilisationsgehäuses mindestens eine Öffnung (3) aufweist, die in Größe oder Gestalt angepasst ist, (um) Radiofrequenzsignalen zu erlauben, in das Gehäuse einzudringen und es zu verlassen.

11. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei die mit dem Instrument assoziierte Radiofrequenzidentifikationsmarkierung innerhalb des Instruments eingebettet ist.

12. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei die mit dem Instrument assoziierte Radiofrequenzidentifikationsmarkierung auf der Außenoberfläche des medizinischen Instruments befestigt ist.

13. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei jede der Radiofrequenzidentifikationsmarkierungen eine passive Vorrichtung ist.

14. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei jede der Radiofrequenzidentifikationsmarkierungen eine aktive Vorrichtung ist.

15. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei das Lesegerät elektrisch mit einem Mikroprozessor verbunden ist.

16. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei das Lesegerät sich in drahtloser Kommunikation mit einem Mikroprozessor (102) befindet.

17. Verfahren zur Identifikation mindestens eines an einer Radiofrequenzidentifikationsmarkierung befestigten oder damit assoziierten medizinischen Instruments in einem Sterilisationsgehäuse in Anspruch 10, wobei die Informationen mindestens eins der Folgendes einschließen:
die Identifikation des medizinischen Instruments, mit dem die Radiofrequenzidentifikationsmarkierung assoziiert ist;
die mit dem medizinischen Instrument, mit dem die Radiofrequenzidentifikationsmarkierung assoziiert ist, assoziierte chirurgische Technik; und
die Herstellungsgeschichte des medizinischen Instruments, mit dem die Radiofrequenzidentifikationsmarkierung assoziiert ist.

18. Verfahren zur Identifikation mindestens eines an einer Radiofrequenzidentifikationsmarkierung befestigten oder damit assoziierten medizinischen Instruments in einem Sterilisationsgehäuse in Anspruch 10, ferner **dadurch gekennzeichnet, dass** eine Gehäuse-Radiofrequenzidentifikationsmarkierung an der Außenseite des Sterilisationsgehäuses befestigt oder damit assoziiert ist, wobei die Gehäuse-Radiofrequenz via Radiofrequenz Informationen von den mit dem medizinischen Instrument assoziierten Radiofrequenzmarkierungen erhält, und die Informationen via Radiofrequenz an das Lesegerät kommuniziert.

## Revendications

1. Un système pour identifier des instruments médicaux dans un boîtier de stérilisation comprenant : (a) au moins une étiquette d'identification radiofréquence (7, 8) : (b) au moins un instrument médical (6) contenu dans un boîtier (1) et attaché à l'étiquette d'identification radiofréquence ; (c) un lecteur (2) conçu pour obtenir des informations, via radiofréquence, depuis ladite étiquette d'identification radiofréquence ; dans lequel le boîtier et les étiquettes RFID sont conçus pour permettre au lecteur d'obtenir des informations, via radiofréquence, lorsque l'instrument médical est contenu dans le boîtier, **caractérisé en ce que** le boîtier est un boîtier de stérilisation entouré d'un emballage scellé (4), et **en ce qu'**au moins une surface du boîtier de stérilisation a au moins une ouverture (3) dont la taille ou la forme sont conçues pour permettre à des signaux radiofréquence d'entrer dans le boîtier et de quitter celui-ci.

2. Un système pour identifier des instruments médicaux dans un boîtier de stérilisation selon la revendication 1, dans lequel l'étiquette d'identification radiofréquence associée à l'instrument est incorporée à l'intérieur de l'instrument.

3. Un système pour identifier des instruments médicaux dans un boîtier de stérilisation selon la revendication 1, dans lequel l'étiquette d'identification radiofréquence associée à l'instrument est attachée sur la surface extérieure de l'instrument médical.

4. Un système pour identifier des instruments médicaux dans un boîtier de stérilisation selon la revendication 1, dans lequel chacune des étiquettes d'identification radiofréquence est un dispositif passif.

5. Un système pour identifier des instruments médicaux dans un boîtier de stérilisation selon la revendication 1, dans lequel chacune des étiquettes d'identification radiofréquence est un dispositif actif.

6. Un système pour identifier des instruments médicaux dans un boîtier de stérilisation selon la revendication 1, dans lequel le lecteur est raccordé de façon électrique à un microprocesseur (102).

7. Un système pour identifier des instruments médicaux dans un boîtier de stérilisation selon la revendication 1, dans lequel le lecteur est en communication sans fil avec un microprocesseur (102).

8. Un système pour identifier des instruments médicaux dans un boîtier de stérilisation selon la revendication 1, dans lequel les informations incluent au moins un des éléments suivants :
l'identification de l'instrument médical auquel est associée l'étiquette d'identification radiofréquence ;
la technique chirurgicale associée à l'instrument médical auquel est associée l'étiquette d'identification radiofréquence ; et
l'historique de fabrication de l'instrument médical auquel est associée l'étiquette d'identification radiofréquence.

9. Un système pour identifier des instruments médicaux dans un boîtier de stérilisation selon la revendication 1, **caractérisé en outre en ce qu'**une étiquette d'identification radiofréquence de boîtier (5) attachée ou associée à l'extérieur du boîtier de stérilisation, dans lequel la radiofréquence de boîtier obtient des informations, via radiofréquence, depuis les étiquettes radiofréquence associées aux instruments médicaux et communique les informations au lecteur via radiofréquence.

10. Une méthode pour identifier au moins un instrument médical (6) attaché ou associé à une étiquette d'identification radiofréquence (7, 8) dans un boîtier (1) avec un lecteur (2) qui obtient des informations depuis l'étiquette d'identification radiofréquence, la méthode comprenant le fait :
de fournir le boîtier configuré pour permettre à des signaux radiofréquence d'entrer dans le boîtier et de quitter celui-ci et d'insérer chaque instrument médical et étiquette d'identification radiofréquence dans le boîtier ; et
d'utiliser le lecteur pour obtenir des informations, via radiofréquence, lorsque chaque instrument médical est contenu dans le boîtier, **caractérisée en ce que** le boîtier est un boîtier de stérilisation entouré d'un emballage scellé (4), et
au moins une surface du boîtier de stérilisation a au moins une ouverture (3) dont la taille et la forme sont conçues pour permettre à des signaux radiofréquence d'entrer dans le boîtier et de quitter celui-ci.

11. Une méthode pour identifier au moins un instrument médical attaché ou associé à une étiquette d'identification radiofréquence dans un boîtier de stérilisation dans la revendication 10, dans laquelle l'étiquette d'identification radiofréquence associée à l'instrument est incorporée à l'intérieur de l'instrument.

12. Une méthode pour identifier au moins un instrument médical attaché ou associé à une étiquette d'identification radiofréquence dans un boîtier de stérilisation dans la revendication 10, dans laquelle l'étiquette d'identification radiofréquence associée à l'instrument est attachée sur la surface extérieure de l'instrument médical.

13. Une méthode pour identifier au moins un instrument médical attaché ou associé à une étiquette d'identification radiofréquence dans un boîtier de stérilisation dans la revendication 10, dans laquelle chacune des étiquettes d'identification radiofréquence est un dispositif passif.

14. Une méthode pour identifier au moins un instrument médical attaché ou associé à une étiquette d'identification radiofréquence dans un boîtier de stérilisation dans la revendication 10, dans lequel chacune des étiquettes d'identification radiofréquence est un dispositif actif.

15. Une méthode pour identifier au moins un instrument médical attaché ou associé à une étiquette d'identification radiofréquence dans un boîtier de stérilisation dans la revendication 10, dans laquelle le lecteur est raccordé de façon électrique à un microprocesseur.

16. Une méthode pour identifier au moins un instrument médical attaché ou associé à une étiquette d'identification radiofréquence dans un boîtier de stérilisation dans la revendication 10, dans laquelle le lecteur est en communication sans fil avec un microprocesseur (102).

17. Une méthode pour identifier au moins un instrument médical attaché ou associé à une étiquette d'identification radiofréquence dans un boîtier de stérilisation dans la revendication 10, dans laquelle les informations incluent au moins un des éléments suivants :
l'identification de l'instrument médical auquel est associée l'étiquette d'identification radiofréquence ;
la technique chirurgicale associée à l'instrument médical auquel est associée l'étiquette d'identification radiofréquence ; et
l'historique de fabrication de l'instrument médical auquel est associée l'étiquette d'identification radiofréquence.

18. Une méthode pour identifier au moins un instrument médical attaché ou associé à une étiquette d'identification radiofréquence dans un boîtier de stérilisation dans la revendication 10, **caractérisée en outre en ce qu'**une étiquette d'identification radiofréquence de boîtier attachée ou associée à l'extérieur du boîtier de stérilisation, dans laquelle la radiofréquence de boîtier obtient des informations, via radiofréquence, depuis les étiquettes de radiofréquence associées aux instruments médicaux et communique les informations au lecteur via radiofréquence.
